# EUROPEAN PATENT APPLICATION

(11) **EP 1 161 941 A1**
(43) Date of publication of application: **12.12.2001**
(21) Application number: 00908070.6
(22) Date of filing: 14.03.2000
(51) Int. Cl.: A61K 9/20, A61K 47/02, A61K 47/12, A61K 47/36

(54) **QUICKLY DISINTEGRATING TABLETS AND PROCESS FOR PRODUCING THE SAME**

(30) Priority: 15.03.1999 JP 6849499
(71) Applicant: Kaken Pharmaceutical Co., Ltd., Tokyo 113-0021 (JP)
(72) Inventor: MATSUMOTO, Keiko, Kaihatsukenkyusho, Fujieda-shi, Shizuoka, 426-0054 (JP); OHKUMA, Moriyuki, Kaihatsukenkyusho, Fujieda-shi, Shizuoka 426-0054 (JP); KATO, Yasutomi, Kaihatsukenkyusho, Fujieda-shi, Shizuoka 426-0054 (JP); OKUDA, Otomo, Kaihatsukenkyusho, Fujieda-shi, Shizuoka 426-0054 (JP)
(74) Representative: HOFFMANN - EITLE
(86) International application number: JP0001549
(87) International publication number: WO0054752

(57) **Abstract**

Powders for surface modification comprising a pharmacologically active ingredient or a pharmacologically active ingredient and a diluent are surface-modified with light silicic anhydride to prepare pharmacologically active ingredient-comprising surface-modified powders having an improved flowability that enables direct tabletting. The thus surface-modified powders are then blended with excipients including partially alphanized starch or crospovidone, and the blend is directly tableted to produce fast disintegrating tablets. Light silicic anhydride is added to the powders for surface modification comprising a pharmacologically active ingredient or a pharmacologically active ingredient and a diluent, in a proportion of approximately 0.1 to 5 wt%, and partially alphanized starch or crospovidone in a proportion of approximately 10 to 60 wt%, based on the total weight of tablet. The thus obtained surface-modified powders comprising a pharmacologically active ingredient exhibit an angle of repose of 42° or less, and the tablets obtained are fast disintegrating tablets capable of rapidly disintegrating when holding water together in the oral cavity. Multilayered surface modification technique using the combination of dry coating with titanium oxide or erythritol having a mean particle size of 3 µm or less in combination with the aforesaid surface modification enables to produce fast disintegrating tablets which solve tabletting problems that might be caused when using a low melting or highly sticky pharmacologically active ingredient. The multilayered surface modification technique further enables to produce fast disintegrating tablets with an improved taste, when using a pharmacologically active ingredient having a bitter or stinging taste.

## Description

### TECHNICAL FIELD

The present invention relates to surface-modified powders comprising a pharmacologically active ingredient and having a good flowability sufficient to enable direct tabletting, a method for preparing the surface-modified powders and use thereof, as well as fast disintegrating tablets, which have a suitable strength and dissolve instantly in the mouth when holding water in the oral cavity, and a method of producing the tablets using the surface-modified powders comprising a pharmacologically active ingredient.

### BACKGROUND ART

Recently, research has been extensively undertaken to develop orally dissolving preparations having an appropriate disintegratability and solubility to make oral medication easy to the elderly people and infants.

According especially to the silver science research conducted by the Japanese Ministry of Health and Welfare, there is an interesting research report entitled "Studies to manufacture new pharmaceutical preparations and new packaging containers most suitable for administration to elderly people" (Masayasu Sugihara, et al., Tokyo Women's Medical College) (Yakuji Nippo, August 22, 1989 issue). The report discloses buccal dissolution type preparations containing polyethylene glycol 1000 as the base that dissolves in the oral cavity, and oleaginous base as the base that melts at the temperature in the oral cavity, which preparations are obtained by filling the pocket of a polyvinyl chloride molding sheet for press through package (PTP) use with a medicated base, and allowing to cool and mold.

Japanese Patent KOKAI (Laid-Open) No. 5-271054 discloses buccal dissolution type tablets having a porous structure with a hardness of 2 to 25 kg and a porosity of about 20 to 80%, which are manufactured by tabletting a composition comprising a pharmacologically active ingredient, a carbohydrate and water in an amount barely sufficient to wet the surface of particles of the carbohydrate.

On the other hand, in certain countries, buccal dissolution type solid preparations, such as Zydis (trade name) from R. P. Scherer (England) etc., are commercially available. The Zydis preparation is manufactured by blending a pharmacologically active ingredient, a polymer, sugar and other ingredients together, dissolving the blend and freeze-drying the solution (Manuf. Chemist., February 36, 1990).

However, since the buccal dissolution type tablets described above are administered by dissolving in the mouth on contact with saliva, these tablets are not necessarily compatible with practical dosing instructions in the actual medical scene, namely, the dosage form of giving a medicament with a cup of water. It is therefore concerned that confusion might be caused in the actual medical scene. Also, 2 or 3 kinds of drugs are simultaneously administered mostly in practical medication and even 5 or 6 kinds of drugs are given all together in some occasion. Then, it tends to occur such a case that only one medicament is taken without water because of its buccal dissolution tablet form but the other medicaments should be taken with water, which also tends to cause confusion.

The aforesaid methods for preparing buccal dissolution type tablets all involve difficulties in tablet preparation via complex procedures including heating, melting, dissolution, freezing and the like. It is thus desired to prepare buccal dissolution type tablets in a simple way, e.g., by dry granulation, direct tabletting, etc.

However, since powdery active ingredients are poorly flowable, dry granulation technique has been used only for well-fluidizing pharmacologically active ingredients. Physically, direct tabletting of ordinary active ingredients with a small bulk density and poor fluidizing properties is considered extremely difficult since the direct tabletting is greatly affected by the powder characteristics and proportion of pharmacologically active ingredients. Very few attempts to improve powder properties of a pharmacologically active ingredient at the final stage of overall synthesis steps have been hitherto made but the purpose was to avoid problems upon tabletting. Actually, high dose preparations of a pharmacologically active ingredient are very rarely manufactured by direct tabletting.

For these reasons, any report to study dry granulation and direct tabletting of buccal dissolution type tablets has not yet been made.

### DISCLOSURE OF INVENTION

Therefore, it is an object of the present invention to provide fast disintegrating tablets, which have a suitable disintegratability and solubility to be disintegrated to an appropriate size when holding water in the oral cavity so that the tablets are neither adhered to nor choked in the throat, and are easy for aged or pediatric patients to take medicaments, which can still maintain a mechanical strength sufficient to resist destruction in the course of manufacture and storage, and which also have an adequate strength to resist crumbling upon administration, for example, when they are taken out of PTP packages.

It is another object of the present invention to provide a method of producing fast disintegrating tablets by dry granulation technique, in which tablets having the aforesaid excellent properties can be produced in a simple fashion, without requiring complex manufacturing procedures.

It is a further object of the present invention to provide surface-modified powders comprising a pharmacologically active ingredient having a good flowability, which enables to produce, by direct tabletting and dry granulation technique, the aforesaid fast disintegrating tablets that are readily taken by aged or pediatric patients and are thus practically useful.

It is a further object of the present invention to provide a method for producing the surface-modified powders comprising a pharmacologically active ingredient with a good flowability.

It is a still further object of the present invention to provide use of surface-modified powders comprising a pharmacologically active ingredient for producing tablets by direct tabletting of the surface-modified powders comprising a pharmacologically active ingredient.

It is a still further object of the present invention to provide a method of producing tablets, which comprises producing tablets by direct tabletting using the surface-modified powders comprising a pharmacologically active ingredient.

In view of the circumstances described above, the present inventors have made extensive investigations to develop tablets of fast disintegration type and found that, when a pharmacologically active ingredient is subjected to surface modification with a surface modifying base material such as light silicic anhydride, etc., the surface-modified powders comprising a pharmacologically active ingredient with a good flowability can be obtained. The inventors have further found that blending of the surface-modified powders comprising a pharmacologically active ingredient with a disintegrant such as partially alphanized starch, crospovidone, etc. followed by direct tabletting provides fast or rapidly disintegrating tablets that maintain an adequate hardness and are yet rapidly disintegrated and dissolved in the oral cavity without resort to complex production procedures such as heating, melting, dissolving, freezing, etc.; these complex procedures are usually required for conventional production steps. Based on the above findings, the present invention has been accomplished.

Therefore, the present invention relates to surface-modified powders comprising a pharmacologically active ingredient having a flowability sufficient to enable direct tabletting, the powders being surface-modified by subjecting powders for surface modification comprising a pharmacologically active ingredient or a pharmacologically active ingredient and a diluent to surface modification with a surface modifying base material.

The present invention further relates to a method of producing pharmacologically active ingredient-comprising surface-modified powders,- which comprises blending powders for surface modification comprising a pharmacologically active ingredient or a pharmacologically active ingredient and a diluent with a surface modifying base material to modify the surface of the powders, whereby the pharmacologically active ingredient-comprising surface-modified powders having flowability sufficient to enable direct tabletting are produced.

The present invention further relates to a fast disintegrating tablet obtained by blending the pharmacologically active ingredient-comprising surface-modified powders described above with a disintegrant and directly tabletting the blend.

The present invention further relates to a method of producing a fast disintegrating tablet, which comprises blending modifying powders comprising a pharmacologically active ingredient or a pharmacologically active ingredient and a diluent with a surface modifying base material to modify the surface of the powders thereby to prepare pharmacologically active ingredient-comprising surface-modified powders having flowability sufficient to enable direct tabletting, then incorporating a disintegrant into the powders, and directly tabletting the mixture to produce the fast disintegrating tablet.

The present invention further relates to use of pharmacologically active ingredient-comprising surface-modified powders for the production of tablets by directly tabletting the pharmacologically active ingredient-comprising surface-modified powders optionally after adding an additive(s) to the powders.

### BEST MODE FOR CARRYING OUT THE INVENTION

The pharmacologically active ingredient for use in the present invention may be in any optional form so long as the active ingredient takes a powdery form at ambient temperature. The active ingredient may also be in a crystalline or non-crystalline form. When pharmacologically active ingredients take no powdery form at ambient temperature, these active ingredients may be converted into a powdery form at ambient temperature by adsorbing these active ingredients onto a porous carrier such as special calcium silicate, starch, etc.

Specifically, the pharmacologically active ingredients for use in the present invention may be at least one member selected from the group consisting of gastrointestinal function conditioning agents, antacids, analgesics, antiinflammatory agents, antiinflammatory-analgesic-antipyretic agents, antibacterial agents, antifungal agents, cardiotonics, inorganics preparations, therapeutic agents for digestive ulcer, coronary vasodilators, peripheral vasodilators and cerebrovasodilators, anti-infectives, anti-anxiety drugs, neuroleptic agents, central nervous system stimulants, antidepressants, antihistamines, antidiarrheal preparations, mild laxatives, nourishing and health-promoting agents, cholesterol lowering agents, spasmolytics, antiagony agents, heart rhythm regulators, therapeutic agents for arterial hypertension, antimigraine agents, blood coagulation regulators, therapeutic agents for thyroid dysfunction, diuretics, appetite suppressants, antiasthmatics, antitussives, expectorants, sedative expectrants, mucus controlling agents, antiemetics, anti-uricemic agents, therapeutic agents for gout, antiarrhythmic drugs, therapeutic drugs for hyperglycemia, bronchodilators, antidiabetic agents, oral contraceptives, therapeutic drugs for travel sickness, therapeutic drugs for prostatic hypertrophy, therapeutic drugs for pancreatitis, hypnotic inducers, hypnotic-sedative agents, antirheumatoid agents, antiepileptics, cerebral metabolism improving agents, anti-platelet drugs, vitamins, and so on.

More specifically, there are antacids such as calcium carbonate, sodium hydrogencarbonate, magnesium oxide, etc.; antiinflammatory agents such as diclofenac sodium, ketoprofen, indomethacin, flurbiprofen, etc.; analgesic-antipyretic agents such as ibuprofen, acetaminophen, caffeine anhydride, aspirin, ethenzamide, tolfenamic acid, mefenamic acid, phenacetin, flufenamic acid, salicylamide, aminopyrine, pentazocine, etc.; antiphlogistic agents such as serrapeptidase, lysozyme chloride, etc.; cardiotonics such as dl-chlorpheniramine maleate, bupranolol hydrochloride, nitroglycerine, isosorbide nitrate, pindolol, propranolol hydrochloride, nifedipine, diltiazem hydrochloride, nisoldipine, benidipine hydrochloride, acebutolol hydrochloride, labetalol hydrochloride, amlodipine besilate, verapamil hydrochloride, etc.; inorganics preparations such as potassium gluconate, calcium gluconate, sodium chloride, potassium chloride, calcium chloride, etc.; therapeutic agents for digestive ulcer such as glycopyrronium bromide, proglumide, butylscopolamine bromide, benactyzine methobromide, propantheline bromide, cimetidine, famotidine, omeprazole, lansoprazole, oxethazaine, roxatidine acetate hydrochloride, L-glutamine, L-glutamate-water soluble azulene, sucralfate, etc.; coronary vasodilators such as phenylpropanolamine hydrochloride, etc.; peripheral and cerebrovasodilators such as beraprost sodium, etc.; antihistamines such as diphenhydramine hydrochloride, etc.; therapeutic drugs for arterial hypertension such as uradipil, etc.; antitussives-sedative expectrants such as dihydrocodeine phosphate, noscapine, methylephedrine hydrochloride, dextromethorphan hydrobromide, etc.; therapeutic drugs for gout such as bucolome, colchicine, probenecid, benzbromarone, sulfinpyrazone, allopurinol, etc.; bronchodilators such as aminophylline, theophylline, etc.; antidiabetic agents such as acetohexamie, glibenclamide, tolazamide, tolbutamide, etc.; therapeutic drugs for pancreatitis such as camostat mesylate, etc.

The active ingredient itself may be employed for the present invention, or may be diluted with a diluent that is used generally in the pharmaceutical or food industry. Examples of such diluents include lactose, anhydrous calcium hydrogenphosphate, crystalline cellulose, sucrose, D-mannitol, low substituted hydroxypropyl cellulose, xylitol, erythritol, trehalose, aspartame, etc. Among them, preferred are lactose, anhydrous calcium hydrogenphosphate and crystalline cellulose.

Where the diluent is employed in the powders, the amount of the diluent to be used has no particular limitation and may vary depending on kind of the pharmacologically active ingredient, but the diluent is used generally at least in a 5-fold amount by weight based on the pharmacologically active ingredient.

According to the present invention, the powders for surface modification comprising the pharmacologically active ingredient described above or comprising the pharmacologically active ingredient and optionally further containing the diluent described above are subjected to surface modification with a surface modifying base material to provide the pharmacologically active ingredient-comprising surface-modified powders having an improved flowability.

Any material can be used as the surface modifying base material of the present invention so long as the material does not adversely affect the pharmacologically active ingredient and can improve flowability. Preferably, the surface modifying base materials are those capable of physically adhering to the surface of the powders for surface modification thereby to contribute to improving flowability of the powders. Examples of such surface modifying base materials include light silicic anhydride, talc, stearic acid, magnesium stearate, calcium stearate, starch, titanium oxide, citric acid, malic acid, adipic acid, hydrous silicon dioxide, calcium carbonate, etc. Preferred are light silicic anhydride, talc, stearic acid, magnesium stearate and calcium stearate, with light silicic anhydride being particularly preferred. Also, the surface modifying base material has preferably a mean particle size of 10 µm or less; if desired, the surface modifying base material may be ground to powders, which may be provided for use as the base material.

For subjecting the surface-modifying powders comprising the aforesaid pharmacologically active ingredient or the active ingredient and the diluent to the surface modification with the surface modifying base material to improve the flowability, the surface-modifying powders are thoroughly blended with the surface modifying base material. More specifically, the blending can be carried out by any of blending devices conventionally used for manufacturing pharmaceutical preparations such as devices for surface modification, high speed mixers, high speed agitation granulators, versatile mixer and so on. Examples of such devices include Mechanomill (manufactured by Okada Seiko Co., Ltd.) Vertical Granulator (manufactured by Powrex), High Speed Mixer (manufactured by Fukae Powtec), Hybridizer, Laboratory Matrix (manufactured by Nara Machinery Co., Ltd.), Theta Composer (manufactured by Tokuju Corporation), etc.

The amount of the surface modifying base material described above may vary depending upon kind of the pharmacologically active ingredient used but generally, the surface modifying base material is incorporated in the obtained active ingredient-comprising surface-modified powders in the amount of approximately 0.1 to 5 wt%, preferably about 1 to about 3 wt%.

In case that the pharmacologically active ingredient having a low melting point or of a sticky property, such as ibuprofen, is used in the present invention, it is preferred to add finely divided titanium oxide or talc powders to the active ingredient before or after the surface modification described above at the following production steps, in order to improve tabletting problems, i.e., sticking or binding in preparing, e.g., flat tablets. Also where a bitter or stinging active ingredient such as ibuprofen is employed in the present invention, finely divided sweeteners such as erythritol or trehalose are advantageously added to the active ingredient, before or after the surface modification, in the same way as described above.

Preferably, the addition of these components is effected by a so-called multiple layer surface modification method, which involves adding these components to the powders comprising the pharmacologically active ingredient or the pharmacologically active ingredient and the diluent before surface modification or to the pharmacologically active ingredient-comprising surface-modified powders after surface modification, and then dry coating the blend with a high speed mixer, a high speed agitation granulator, a general-purpose kneader, etc. as described above.

Preferably, the components of finely divided titanium oxide, talc, erythritol, trehalose, etc. described above have a mean particle size of 3 µm or less, and the amount of the finely divided components is in the range of 3 wt% or less, based on the total weight of the pharmacologically active surface-modified powders finally obtained.

The thus obtained pharmacologically active ingredient-comprising surface-modified powders generally have a particle size of approximately 20 to 200 µm, preferably about 40 to about 110 µm.

Where a pharmacologically active ingredient requiring only a small dose is used to produce the pharmacologically active ingredient-comprising surface-modified powders of the present invention, the following procedures are advantageously employed.

For example, when the pharmacologically active ingredient-comprising surface-modified powders are designed to contain 0.02 to 10 wt% of the active ingredient, a diluent such as lactose is added in the course of surface modification to produce the pharmacologically active ingredient-comprising surface-modified powders. Where lactose is used, large particles of lactose for direct tabletting may be used. Suitable examples of the pharmacologically active ingredient used to produce such pharmacologically active ingredient-comprising surface-modified powders are glycopyrronium bromide, bupranolol hydrochloride and beraprost sodium.

Unless the effects contemplated in the invention are interfered with, the surface-modified powders may further contain a variety of additives, which are commonly used in the manufacture of pharmaceutical preparations. Specific examples of such additives will be described hereinafter.

The pharmacologically active ingredient-comprising surface-modified powders of the present invention described above in detail have an excellent flowability. The surface-modified powders of the present invention exhibit flowability at an angle of repose of 42° or less, and more preferably, at an angle of repose of 40° or less. Therefore, the pharmacologically active ingredient-comprising surface-modified powders enable to produce tablets by dry procedures using direct tabletting. Thus, the powders can be used not only as substrates for producing the fast disintegrating tablets of the present invention described in the following section, but also as substrates for preparing tablets by dry procedures using other conventional direct tabletting. These tablets may also be produced by, for example, admixing the pharmacologically active ingredient-comprising surface-modified powders of the present invention with conventional additives, and tabletting the mixture with a direct tabletting machine.

According to the present invention, by admixing the pharmacologically active ingredient-comprising surface-modified powders and a disintegrant and directly tabletting the mixture, the fast disintegrating tablets of the present invention can be produced. The fast disintegrating tablet of the invention have an appropriate disintegratability and solubility, are disintegrated into a suitable size when holding water in the oral cavity to cause no stick or choking to the throat and thus make oral medication easy to aged or pediatric patients.

The pharmacologically active ingredient-comprising surface-modified powders may be used as admixture of a plurality of pharmacologically active ingredient-comprising surface-modified powders using different active components.

Specific examples of the disintegrants used in the present invention include partially alphanized starch, crospovidone (Polyplasdone), crystalline cellulose-carmellose sodium, low substituted hydroxypropyl cellulose, corn starch, potato and other starches, carmellose, carmellose calcium, carmellose sodium, croscarmellose sodium, carboxymethylstarch sodium, etc. Among them, partially alphanized starch and crospovidone are particularly preferred.

The amount of the disintegrant which is mixed with the pharmacologically active ingredient-comprising surface-modified powders may vary depending on kind of the active ingredient used, but the disintegrant is used in an amount of generally 10 to 60 wt%, preferably about 20 to 40 wt%, based on the weight of the tablet finally obtained.

When a smaller dose of the active ingredient is required as in therapeutic agents for angina pectoris, therapeutic drugs for digestive ulcer, peripheral vasodilators, etc. or a moderate dose is required as for antiinflammatory-analgesic-antipyretic agents, the disintegrant is added in the amount of generally 10 to 60 wt%, and preferably about 20 to about 40%, based on the total weight of the tablet finally obtained.

When inorganics or the like requiring a large dose are used as the pharmacologically active ingredient, the disintegrant is added in the amount of generally 10 to 40 wt%, and preferably about 20 to about 30%, based on the total weight of the tablet finally obtained.

In addition to the disintegrant described above, the surface-modified powders may further contain a variety of additives conventionally employed in the manufacture of tablets, unless the effects of the invention are interfered with these additives.

Such additives include, for example, binders, acids, foaming agents, artificial sweeteners, flavorings, lubricants, colorants and so on.

Examples of the binders include hydroxypropylcellulose, hydroxypropylmethylcellulose 2910, hydroxypropyl starch, gelatin, pullulan and the like. Examples of the acids include citric acid, tartaric acid, malic acid, adipic acid and so on. Examples of the foaming agents include citric acid, sodium bicarbonate and so on. Examples of the artificial sweeteners include saccharin sodium, glycyrrhizic acid dipotassium salt, aspartame, stevia, thaumatin, xylitol, erythritol, glucose, and so on. Examples of the flavorings include lemon, orange, grape fruit, grape, menthol, spearmint, peppermint, vanilla, cinnamon, and the like. Examples of the lubricants include magnesium stearate, talc, sucrose fatty acid esters, polyethyleneglycol, stearic acid and the like. Examples of the colorants include various food colorants, e.g. FD & C Yellow No. 5, FD & C RED No.2, FD & C Blue No.2, etc., food lakes, red iron oxide and so on.

One or more of these additives can be timely added in appropriate proportions, for example, at any step in the course of blending or before and after blending the pharmacologically active ingredient-comprising surface-modified powders with the disintegrant.

In the present invention, it is advantageous to use, among others, crystalline cellulose (trade name: CEOLUS, manufactured by Asahikasei Corporation) as the additive, in combination with the disintegrant, since the hardness of the tablets obtained is increased. Where crystalline cellulose is added to the powders, the hardness of the resulting tablet is enhanced, while the tablet retains its rapid disintegrating property. The crystalline cellulose is added to the powders to have a proportion of 10 to 70 wt%, based on the total weight of the tablet finally obtained.

The blending of the pharmacologically active ingredient-comprising surface-modified powders with the disintegrant and optionally additives can be carried out by any blending techniques such as mixing, kneading, sieving and so on, which are conventionally used in the course of manufacturing pharmaceutical preparations. Specifically, a V-Shaped Mixer, Gyro Shifter, Theta Composer (manufactured by Tokuju Corporation), Vertical Granulator (manufactured by Powrex), High Speed Mixer (manufactured by Fukae Powtec), Laboratory Matrix (manufactured by Nara Machinery Co., Ltd.), etc. can be employed.

For tabletting the blend or mixture thus obtained, devices conventionally used for molding of tablets can be employed. For example, a single tabletting machine, a rotary tabletting machine, a full-automated rotary tabletting machine (manufactured by Hata Iron Works), etc. are available.

In tabletting with the above tabletting machines, a compression pressure greatly changes depending on tablet size. When using a pestle of 8 mm in diameter, the compression pressure is generally set under 190 to 1,990 Kg/cm², with a pestle of 10 mm in diameter, generally under 250 to 1,920 Kg/cm², and with a pestle of 20 mm in diameter, generally under 520 to 1,320 Kg/cm². The tablet is compression-molded to have an appropriate strength well resistant to the steps of storage, packaging and transportation.

As described above, the fast disintegrating tablets of the present invention can be extremely readily produced by dry procedures using production devices conventionally available, without requiring any complex step. Moreover, the thus produced fast disintegrating tablets are excellent in solubility and disintegratability.

The fast disintegrating tablets of the present invention have a suitable disintegratability and solubility to be disintegrated to a desired size when holding water in the oral cavity so that the tablets are neither adhered to nor choked in the throat, and are easy for administration to aged or pediatric patients. Accordingly, the tablets of the present invention can be advantageously used for the treatment and prevention of diseases in patients, particularly aged or pediatric patients.

Furthermore, the fast disintegrating tablets of the present invention are excellent in storage and stability over a long period of time, because the tablets possess a desired strength. The tablets of the present invention retain a mechanical strength sufficient to resist destruction when the tablets are taken by the patient, for example, when they are taken out of PTP packages. Therefore, the fast disintegrating tablets of the invention can be advantageously used for the patient who should be medicated depending on the active ingredient contained, for the treatment and prevention of diseases in patients, particularly aged or pediatric patients.

Hereinafter, the present invention will be described in more detail, with reference to EXAMPLES but is not deemed to be limited thereto.

### EXAMPLE 1

A high speed agitation granulator (Laboratory Matrix LMA10, manufactured by Nara Machinery Co., Ltd.) was charged with 2 parts by weight of light silicic anhydride based on 100 parts by weight of ibuprofen to examine the conditions for surface modification. A mean particle size became constant when the time for surface modification showed 15 minutes. Measurement of the angle of repose 25 minutes after revealed that flowability was markedly improved. It was thus confirmed that the powders containing the original drug for direct tabletting, namely, the pharmacologically active ingredient-comprising surface-modified powders of the present invention were obtained. The procedures and results are shown in TABLE 1.

**TABLE 1**

| Time for Surface Modification (min.) | Mean Particle Size (µm)^{*1} | Angle of Repose (°)^{*2} |
|---|---|---|
| 0 | 50.5 | 53 |
| 3 | 46.1 | - |
| 5 | 48.0 | 45 |
| 10 | 55.8 | 42 |
| 15 | 57.7 | 40 |
| 20 | 57.2 | 39 |
| 25 | 57.3 | 39 |
| Conditions for surface modification: | | |
| main impeller: 300 rpm | | |
| granulation impeller: 1,500 rpm | | |

| | | |
|---|---|---|
| *1: The mean particle size was measured using a Laser diffraction size analyzer LDSA-1400A (manufactured by Tohnichi Computer Application Co., Ltd.). | | |
| *2: The angle of repose was measured with a powder tester (manufactured by Hosokawa Micron Co., Ltd.). | | |

### EXAMPLE 2

A high speed agitation granulator (Laboratory Matrix LMA10, manufactured by Nara Machinery Co., Ltd.) was charged with 1 part by weight of light silicic anhydride based on 100 parts by weight of potassium gluconate to examine the conditions for surface modification in the same manner as in EXAMPLE 1. The results are shown in TABLE 2.

**TABLE 2**

| Time for Surface Modification (min.) | Mean Particle Size (µm) | Angle of Repose (°) |
|---|---|---|
| 0 | 111.3 | 41 |
| 5 | 123.6 | 39 |
| 10 | 122.5 | 37 |
| 15 | 121.5 | 37 |
| Conditions for surface modification: main impeller: 300 rpm granulation impeller: 1,500 rpm Measurement of the mean particle size and angle of repose: | | |
| The same devices as in EXAMPLE 1 were used. | | |

The results of EXAMPLES 1 and 2 reveal that by adding light silicic anhydride to the pharmacologically active ingredient to effect the surface modification, the flowability (angle of repose) of the pharmacologically active ingredient-comprising surface-modified powders of the present invention was markedly improved to give the flowability sufficient to perform direct tabletting continuously. The results further reveal that the time required for surface modification was at least 5 minutes, preferably 10 to 20 minutes.

Furthermore, the time required for surface modification can be judged by the time when the angle of repose becomes 40° or less.

The results of EXAMPLES 1 and 2 reveal that when the active ingredient has a particle size of 20 µm or more, the surface-modified product with a good quality, i.e., the pharmacologically active ingredient-comprising surface-modified powders of the invention can be obtained, since the mean particle size of ibuprofen and potassium gluconate were found to be 50.5 µm (10% particle size: 19.2 µm, 90% particle size: 101.6 µm) and 111.3 µm, respectively. The pharmacologically active ingredient of at least 50 µm is preferred for use in the present invention.

### EXAMPLE 3

A high speed agitation granulator (Laboratory Matrix LMA10, manufactured by Nara Machinery Co., Ltd.) was charged with ibuprofen and light silicic anhydride in the respective amounts shown in TABLE 3 below. Surface modification was performed for 25 minutes (conditions for the surface modification: a main impeller of 300 rpm, a granulation impeller of 1,500 rpm). Next, the whole amount of the pharmacologically active ingredient-comprising surface-modified powders of the present invention obtained above were taken out of the granulator. The remaining components given in TABLE 3 below, i.e., citric acid, partially alphanized starch (manufactured by Asahikasei Corporation, trade name: PCS), crystalline cellulose (manufactured by Asahikasei Corporation, Avicel PH301) and magnesium stearate were added to the powders, followed by mixing with a V-10 blender (manufactured by Tokuju Corporation) for 5 minutes. The mixture was tableted with a pestle of 10 mm in diameter and 7.5 mmR using a fully automated rotary tabletting machine (manufactured by Hata Iron Works). Thus, the fast disintegrating tablets of the present invention were produced. Flavorings, sweeteners and so on may be incorporated to improve the taste.

**TABLE 3**

| Formulation | |
|---|---|
| Component | Amount Added (unit: g) |
| Ibuprofen | 800 |
| Light silicic anhydride | 16 |
| Citric acid | 200 |
| Partially alphanized starch | 956 |
| Crystalline cellulose | 380 |
| Magnesium stearate | 48 |
| Total | 2400 |

### EXAMPLE 4

The same formulation as in EXAMPLE 3 was surface-modified under the same conditions as those of EXAMPLE 3 and the experiment was carried out in the same way, except that the disintegrant was changed to crospovidone (the amount was the same). A high speed agitation granulator (Laboratory Matrix LMA10, manufactured by Nara Machinery Co., Ltd.) was charged with ibuprofen and light silicic anhydride in the respective amounts indicated in TABLE 4 below. Surface modification was performed for 25 minutes (conditions for the surface modification: main impeller of 300 rpm, granulation impeller of 1,500 rpm). Next, the whole amount of the pharmacologically active ingredient-comprising surface-modified powders of the present invention obtained above were taken out of the granulator. The remaining components given in TABLE 4 below, i.e., citric acid, crospovidone (manufactured by ISP Japan, trade name: Polyplasdone XL), crystalline cellulose and magnesium stearate were added to the powders, followed by mixing with a V-10 blender (manufactured by Tokuju Corporation) for 5 minutes. The mixture was tableted with a pestle of 10 mm in diameter and 7.5 mmR using a fully automated rotary tabletting machine (manufactured by Hata Iron Works). Thus, the fast disintegrating tablets of the present invention were produced. Flavorings, sweeteners and so on may be incorporated to improve taste.

**TABLE 4**

| Formulation | |
|---|---|
| Component | Amount Added (unit: g) |
| Ibuprofen | 800 |
| Light silicic anhydride | 16 |
| Citric acid | 200 |
| Crospovidone | 956 |
| Crystalline cellulose | 380 |
| Magnesium stearate | 48 |
| Total | 2400 |

### EXAMPLE 5

A high speed agitation granulator (Laboratory Matrix LMA10, manufactured by Nara Machinery Co., Ltd.) was charged with potassium gluconate and light silicic anhydride in the respective amounts indicated in TABLE 6 below. Surface modification was performed for 15 minutes (conditions for the surface modification: main impeller of 300 rpm, granulation impeller of 1,500 rpm). Next, the whole amount of the pharmacologically active ingredient-comprising surface-modified powders of the present invention obtained above were taken out of the granulator. The remaining components given in TABLE 5 below, i.e., partially alphanized starch, glucono delta lactone, erythritol, crystalline cellulose and magnesium stearate were added to the powders, followed by mixing with a V-10 blender (manufactured by Tokuju Corporation) for 5 minutes. The mixture was then tableted with a pestle for odd-shaped tablets (16 mm × 7 mm) using a fully automated rotary tabletting machine (manufactured by Hata Iron Works). Thus, the fast disintegrating tablets of the present invention were produced. Flavorings, sweeteners and so on may be incorporated to improve taste.

**TABLE 5**

| Formulation | |
|---|---|
| Component | Amount Added (unit: g) |
| Potassium gluconate | 500 |
| Light silicic anhydride | 5 |
| Partially alphanized starch | 215 |
| Glucono delta lactone | 12 |
| Erythritol | 50 |
| Crystalline cellulose | 100 |
| Magnesium stearate | 18 |
| Total | 900 |

### EXAMPLE 6

A high speed agitation granulator (Laboratory Matrix LMA10, manufactured by Nara Machinery Co., Ltd.) was charged with potassium gluconate and light silicic anhydride in the respective amounts indicated in TABLE 6 below. Surface modification was performed for 15 minutes (conditions for the surface modification; main impeller of 300 rpm, granulation impeller of 1,500 rpm). Next, the whole amount of the pharmacologically active ingredient-comprising surface-modified powders of the present invention obtained above were taken out of the granulator. The remaining components given in TABLE 6 below, i.e., crospovidone, glucono delta lactone, crystalline cellulose and magnesium stearate were added to the powders, followed by mixing with a V-10 blender (manufactured by Tokuju Corporation) for 5 minutes. The mixture was then tableted with a pestle for odd-shaped tablets (19 mm × 8 mm) using a fully automated rotary tabletting machine (manufactured by Hata Iron Works). Thus, the fast disintegrating tablets of the present invention were produced. Flavorings, sweeteners and so on may be incorporated to improve taste.

**TABLE 6**

| Formulation | |
|---|---|
| Component | Amount Added (unit: g) |
| Potassium gluconate | 1000 |
| Light silicic anhydride | 10 |
| Crospovidone | 238 |
| Glucono delta lactone | 24 |
| Crystalline cellulose | 100 |
| Magnesium stearate | 28 |
| Total | 1400 |

### EXAMPLE 7

A high speed agitation granulator (Vertical Granulator FM-VG-01, manufactured by Powrex) was charged with bupranolol hydrochloride and lactose G. After blending with a main impeller and a granulation impeller, both at 500 rpm for 1 minute, 2 wt% of light silicic anhydride was added and surface modification was conducted for 10 minutes under the same conditions. The remaining components given in TABLE 7 below, i.e., partially alphanized starch and magnesium stearate were added to the powders, followed by blending a Transparent Micro V-Mixer (manufactured by Tsutsui Scientific Instruments Co., Ltd.) for 15 minutes. The mixture was then tableted with a flat-faced beveled edged pestle of 8.5 mm in diameter using a fully automated rotary tabletting machine (manufactured by Hata Iron Works). Thus, the fast disintegrating tablets of the present invention were produced. Flavorings, sweeteners and so on may be incorporated to improve taste.

**TABLE 7**

| Formulation | |
|---|---|
| Component | Amount Added (unit: g) |
| Bupranolol hydrochloride | 10 |
| Lactose G | 107.6 |
| Light silicic anhydride | 2.4 |
| Partially alphanized starch | 89.2 |
| Magnesium stearate | 0.8 |
| Total | 210 |

### EXAMPLE 8

A high speed agitation granulator (Vertical Granulator FM-VG-01, manufactured by Powrex) was charged with glycopyrronium bromide and lactose (manufactured by Freund Industrial Co., Ltd., trade name: DAI-LACTOSE S). After blending with a main impeller and a granulation impeller, both at 500 rpm, for 1 minute, 1 wt% of light silicic anhydride was added and surface modification was conducted for 15 minutes under the same conditions. The remaining components shown in TABLE 8 below, i.e., crystalline celluose, partially alphanized starch, erythritol and magnesium stearate were added to the powders, followed by blending a Transparent Micro V-Mixer (manufactured by Tsutsui Scientific Instruments Co., Ltd.) for 15 minutes. The mixture was then tableted with a pestle of 8.5 mm in diameter and 6.5R using a fully automated rotary tabletting machine (manufactured by Hata Iron Works). Thus, the fast disintegrating tablets of the present invention were produced. Flavorings, sweeteners and so on may be incorporated to improve taste.

**TABLE 8**

| Formulation | |
|---|---|
| Component | Amount Added (unit: g) |
| Glycopyrronium bromide | 1 |
| Lactose | 99 |
| Light silicic anhydride | 1 |
| Crystalline cellulose | 30 |
| Partially alphanized starch | 100 |
| Erythritol | 16.5 |
| Magnesium stearate | 2.5 |
| Total | 250 |

### EXAMPLE 9

1) A high speed agitation granulator (Vertical Granulator FM-VG-01, manufactured by Powrex) was charged with ibuprofen and 2 wt% of light silicic anhydride based on ibuprofen. Surface modification was conducted for 25 minutes under the following conditions: a main impeller at 500 rmp and a granulation impeller at 500 rpm.
2) Next, a high speed agitation granulator (Vertical Granulator FM-VG-01, manufactured by Powrex) was charged with acetaminophen and 2 wt% of light silicic anhydride based on acetaminophen. Surface modification was conducted for 25 minutes under the following conditions: a main impeller at 500 rmp and a granulation impeller at 500 rpm.
3) Next, a high speed agitation granulator (Vertical Granulator FM-VG-01, manufactured by Powrex) was charged with anhydrous caffeine and 2 wt% of light silicic anhydride based on anhydrous caffeine. Surface modification was conducted for 25 minutes under the following conditions: a main impeller at 500 rmp and a granulation impeller at 500 rpm.
4) The remaining components shown in TABLE 9 below, i.e., citric acid, partially alphanized starch, lactose, crystalline celluose and magnesium stearate were added to the respective three pharmacologically active ingredient-comprising surface-modified powders, followed by blending a V-10 blender (manufactured by Tokuju Corporation) for 5 minutes. Each mixture was then tableted with a pestle of 10 mm in diameter and 7.5 mmR using a fully automated rotary tabletting machine (manufactured by Hata Iron Works). Thus, the fast disintegrating tablets of the present invention were produced. Flavorings, sweeteners and so on may be incorporated to improve taste.

**TABLE 9**

| Formulation | |
|---|---|
| Component | Amount Added (unit: g) |
| Ibuprofen | 75 |
| Acetaminophen | 150 |
| Anhydrous caffeine | 60 |
| Light silicic anhydride | 5.7 |
| Citric acid | 20 |
| Partially alphanized starch | 232.3 |
| Lactose | 100 |
| Crystalline cellulose | 50 |
| Magnesium stearate | 7 |
| Total | 700 |

### TEST EXAMPLE 1

In order to explain the effects achieved by the present invention in more detail, tablet characteristics were determined with the fast disintegrating tablets of the invention obtained in EXAMPLES and commercial products, and comparison was made between the tablets of the invention and the commercial products. The results are shown in TABLES 10 and 11. It is understood by comparing TABLES 10 and 11 that the fast disintegrating tablets obtained in the present invention are excellent in disintegratability and yet maintain a suitable strength. It is also confirmed that partially alphanized starch and crospovidone were most suitable as the disintegrant.

### Characteristic data of the experimental tablets prepared in EXAMPLES:

**TABLE 10**

| No. Example | 1-1 3 | 1-2 4 | 2-1 5 | 2-2 6 | 3 7 | 4 8 | 5 9 |
|---|---|---|---|---|---|---|---|
| Weight (mg) | 300 | 300 | 900 | 1400 | 210 | 250 | 350 |
| Thickness of tablet (mm) | 5.4 | 5.2 | 6.2 | 9.2 | 5.1 | 6.0 | 5.8 |
| Diameter of tablet (mn) | 10.1 | 10.1 | 16×7 | 19×8 | 8.6 | 8.6 | 10.1 |
| Hardness (kg) | 4.0 | 6.0 | 4.8 | 6.1 | 3.1 | 2.7 | 5.3 |
| Disintegration time (1) | 29 secs. | 15 secs. | 120 secs. | 29 secs. | 45 secs. | 30 secs. | 34 secs. |
| Disintegration time (2) | 62 secs. | 37 secs. | 186 secs. | 120 secs. | 79 secs. | 30 secs. | 58 secs. |

### Hardness:

The hardness is expressed as a mean value of five measurements as measured with Kiya type Hardness Tester.
(Hardness tester: HARDNESS TESTER (manufactured by KIYA))

### Disintegration time (1):

The disintegration time was measured with a disintegration tester specified in The Pharmacopoeia of Japan, 13th edition (no disk). The results indicate a mean value of six runs.

(Disintegration tester: table disintegration
tester T-4H (manufactured by Toyama Sangyo Co., Ltd.))

### Disintegration time (2):

A sieve of 5.5 mesh was put in a beaker charged with 300 mL of water. One tablet was placed in the beaker. The total elapsed time required for the tablet to disintegrate, completely pass through the sieve and fall out of the mesh was recorded (distilled water: 23°C). The disintegration time is the average of the time recorded for 2 runs.

### Characteristic data of the commercial products:

**TABLE 11**

| No. of Commercial Tablet | Tablet 1 | Tablet 2 | Tablet 3 | Tablet 4 |
|---|---|---|---|---|
| Preparation form | Dragee | Film-coated | Film-coated | Uncoated |
| Weight (mg) | 224 | 1245 | 178 | 203 |
| Thickness of tablet (mm) | 4.8 | 7.6 | 4.5 | 2.7 |
| Diameter of tablet (mm) | 8.1 | 19×8 | 7.7 | 8.6 |
| Hardness (kg) | 6.9 | ≧20 | 9.1 | 4.5 |
| Disintegration time (1) | 10 mins. | ≧30 mins. | 3 mins. | 1 min. |
| Disintegration time (2) | ≧30 mins. | ≧30 mins. | 7 mins. | 80 secs. |

### Hardness:

The hardness is expressed as a mean value of five measurements as measured with Kiya type Hardness Tester.
(Hardness tester: HARDNESS TESTER (manufactured by KIYA))

### Disintegration time (1):

The disintegration time was measured with a disintegration tester specified in The Pharmacopoeia of Japan, 13th edition (no disk). The results indicate a mean value of six runs.
(Disintegration tester: table disintegration tester T-4H (manufactured by Toyama Sangyo Co., Ltd.))

### Disintegration time (2):

A sieve of 5.5 mesh was put in a beaker charged with 300 mL of water. One tablet was placed in the beaker. The total elapsed time required for the tablet to disintegrate, completely pass through the sieve and fall out of the mesh was recorded (distilled water: 23°C). The disintegration time is the average of the time recorded for 2 runs.

### EXAMPLE 10

The blended powders produced in EXAMPLE 3 were tableted with a pestle having flat-faced beveled edges of 10 mm in diameter. Tabletting problem (sticking to the pestle) was observed.

The material stuck to the pestle was found to be ibuprofen. It was considered to be caused by difference in shape of the pestle.

In order to solve the tabletting problem, titanium oxide (manufactured by Toho Titanium K.K., trade name: Highly Pure Titanium Oxide) was coated onto the surface of the surface-modified powders to form multiple layers. Thus, the fast disintegrating tablets could be produced.

The procedures for producing the fast disintegrating tablets are described below.

A high speed agitation granulator (Laboratory Matrix LMA10, manufactured by Nara Machinery Co., Ltd.) was charged with ibuprofen and light silicic anhydride in the respective amounts shown in TABLE 12 below. Surface modification was performed for 25 minutes (conditions for the surface modification: a main impeller of 300 rpm, a granulation impeller of 1,500 rpm). Next, titanium oxide having a mean particle size of 2.1 µm was added as indicated in TABLE 12 below to perform dry coating for 5 minutes under the same conditions. Thus, the multilayered surface-modified powders with titanium oxide coating on the surface of the powders were prepared.

Thereafter, the whole amount of the pharmacologically active ingredient-comprising surface-modified powders of the present invention obtained above were taken out of the granulator. The remaining components given in TABLE 12 below, i.e., citric acid (manufactured by Wako Pure Chemical Industries Ltd.), partially alphanized starch (manufactured by Asahikasei Corporation), crystalline cellulose (manufactured by Asahikasei Corporation, CEOLUS), talc (manufactured by Shokozan Mining Co., Ltd.) and magnesium stearate (manufactured by Kyodo Yakuhin K.K.) were added to the powders, followed by mixing with a V-10 blender (manufactured by Tokuju Corporation) for 5 minutes. By tabletting the mixture with a pestle of 10 mm in diameter having flat-faced beveled edges using a tabletting machine, the tabletting problem was eliminated. Thus, the fast disintegrating tablets of the present invention were produced. The results indicate that by the multilayered surface modifying procedures, there could be obtained the fast disintegrating tablets available for low melting or strongly sticky drugs, irrespective of the shape of a pestle.

**TABLE 12**

| Formulation | |
|---|---|
| Component | Amount Added (unit: g) |
| Ibuprofen | 800 |
| Light silicic anhydride | 8 |
| Titanium oxide | 24 |
| Citric acid | 136 |
| Partially alphanized starch | 956 |
| Crystalline cellulose | 380 |
| Magnesium stearate | 48 |
| Talc | 48 |
| Total | 2400 |

### EXAMPLE 11

Taste comparison was performed with the tablets prepared in EXAMPLE 10. A strongly stinging taste spread over the entire tongue. The irritating component was found to be ibuprofen.

Therefore, an attempt to mask the stinging taste of the ibuprofen-containing fast disintegrating tablets and change them to compatible medicaments easy to be taken was made to alleviate the irritation of a drug particle carrier by multilayered surface modifying technique using the surface modification in combination with dry coating as in EXAMPLE 10. Finely divided erythritol (mean particle size: 1.3 µm) was used to reduce the stinging taste of ibuprofen. Erythritol was again added to the powders at the final stage to improve the taste.

The procedures of making pharmaceutical preparations with an improved taste and further functioning as a fast disintegrating tablet are described below.

A high speed agitation granulator (Laboratory Matrix LMA10, manufactured by Nara Machinery Co., Ltd.) was charged with ibuprofen and light silicic anhydride in the respective amounts shown in TABLE 13 below. Surface modification was performed for 25 minutes (conditions for the surface modification: a main impeller of 300 rpm, a granulation impeller of 1,500 rpm). Next, titanium oxide having a mean particle size of 2.1 µm and as a sweetener, erythritol finely divided with a jet mill in a mean particle size of 1.3 µm were added as indicated in TABLE 13 below to perform dry coating for 5 minutes under the same conditions. Thus, the multilayered surface-modified powders, on the surface of which titanium oxide and jet mill-grounded erythritol had been coated were prepared.

Then, the whole amount of the pharmacologically active ingredient-comprising surface-modified powders of the present invention obtained above were taken out of the granulator. The remaining components given in TABLE 13 below, namely, citric acid (manufactured by Wako Pure Chemical Industries Ltd.), partially alphanized starch (manufactured by Asahikasei Corporation), crystalline cellulose (manufactured by Asahikasei Corporation, CEOLUS), talc (manufactured by Shokozan Mining Co., Ltd.), erythritol (manufactured by Nikken Chemical Industry Co., Ltd.), a flavoring agent (manufactured by Ogawa Perfumery Co., Ltd.) and magnesium stearate (manufactured by Kyodo Yakuhin K.K.) were added to the powders, followed by blending with a V-10 blender (manufactured by Tokuju Corporation) for 5 minutes. Any tabletting problem that might be caused by tabletting the blend with a pestle of 10 mm in diameter having flat-faced beveled edges using a tabletting machine was not noted. Moreover, the thus produced fast disintegrating tablets of the present invention were improved on the stinging taste. The results indicate that by the multilayered surface modifying technique, the fast disintegrating tablets were found to be available for improving the taste of drugs having a bitter or stinging taste.

**TABLE 13**

| Formulation | |
|---|---|
| Component | Amount Added (unit: g) |
| Ibuprofen | 400 |
| Light silicic anhydride | 4 |
| Titanium oxide | 4 |
| Finely divided erythritol | 12 |
| Citric acid | 40 |
| Erythritol | 400 |
| Partially alphanized starch | 480 |
| Crystalline cellulose | 1000 |
| Flavoring agent (orange) | 12 |
| Magnesium stearate | 24 |
| Talc | 24 |
| Total | 2400 |

### EXAMPLE 12

It was examined if the order of the multilayered surface modification technique used in EXAMPLE 12, namely, the surface modification and dry coating, would affect the tablet characteristics. For the comparison purpose, the same formulation as that of EXAMPLE 11 was used in this EXAMPLE.

The procedures are described below.

A high speed agitation granulator (Laboratory Matrix LMA10, manufactured by Nara Machinery Co., Ltd.) was charged with ibuprofen, titanium oxide having a mean particle diameter of 2.1 µm and finely divided erythritol (a mean particle size: 1.3 µm) in the respective amounts shown in TABLE 14 below. Dry coating was performed for 5 minutes (a main impeller of 300 rpm, a granulation impeller of 1,500 rpm).

Subsequently, light silicic anhydride was added to the blend as indicated in TABLE 14 below. Surface modification was performed for 25 minutes under the same conditions to produce the multilayered surface-modified powders containing the anti-adhesion agent and the sweetener.

Next, the whole amount of the pharmacologically active ingredient-comprising surface-modified powders of the present invention obtained above were taken out of the granulator. The remaining components given in TABLE 14 below, namely, citric acid (manufactured by Wako Pure Chemical Industries Ltd.), partially alphanized starch (manufactured by Asahikasei Corporation), crystalline cellulose (manufactured by Asahikasei Corporation, CEOLUS), talc (manufactured by Shokozan Mining Co., Ltd.), erythritol (manufactured by Nikken Chemical Industry Co., Ltd.), a flavoring agent (manufactured by Ogawa Perfumery Co., Ltd.) and magnesium stearate (manufactured by Kyodo Yakuhin K.K.) were added to the powders, followed by blending with a V-10 blender (manufactured by Tokuju Corporation) for 5 minutes. The blend was tableted with a pestle of 10 mm in diameter having flat-faced beveled edges using a tabletting machine but no tabletting problem was noted. Moreover, the thus produced fast disintegrating tablets of the present invention had a quality equivalent to those obtained in EXAMPLE 11 in terms of improvement in the stinging taste. The results indicate that by the multilayered surface modifying technique, the fast disintegrating tablets were found to be available not only for low melting or strongly sticky drugs but also for improving the taste of drugs having a bitter or stinging taste. It was confirmed that even if the order of adding the surface modifiers is changed, the tablet preparations can be obtained with an equivalent quality.

**TABLE 14**

| Formulation | |
|---|---|
| Component | Amount Added (unit: g) |
| Ibuprofen | 400 |
| Titanium oxide | 4 |
| Finely divided erythritol | 12 |
| Light silicic anhydride | 4 |
| Citric acid | 40 |
| Erythritol | 400 |
| Partially alphanized starch | 480 |
| Crystalline cellulose | 1000 |
| Flavoring agent (orange) | 12 |
| Magnesium stearate | 24 |
| Talc | 24 |
| Total | 2400 |

### EXAMPLE 13

Using trehalose instead of the sweetener erythritol used in EXAMPLES 11 and 2, it was examined if kind of the sweetener would affect the tablet characteristis. The multilayered surface modifying technique, namely, the order and formulation of the surface modification and the dry coating were the same as in EXAMPLE 12.

The procedures are described below.

A high speed agitation granulator (Laboratory Matrix LMA10, manufactured by Nara Machinery Co., Ltd.) was charged with ibuprofen, titanium oxide having a mean particle diameter of 2.1 µm and finely divided trehalose (a mean particle size: 1.4 µm) (manufactured by Asahikasei Corporation) in the respective amounts shown in TABLE 15 below. Dry coating was performed for 5 minutes (a main impeller of 300 rpm, a granulation impeller of 1,500 rpm).

Subsequently, light silicic anhydride was added to the blend as indicated in TABLE 15 below. Surface modification was performed for 25 minutes under the same conditions to produce the multilayered surface-modified powders containing the anti-adhesion agent and the sweetener.

Next, the whole amount of the pharmacologically active ingredient-comprising surface-modified powders of the present invention obtained above were taken out of the granulator. The remaining components given in TABLE 15 below, namely, citric acid (manufactured by Wako Pure Chemical Industries Ltd.), partially alphanized starch (manufactured by Asahikasei Corporation), crystalline cellulose (manufactured by Asahikasei Corporation, CEOLUS), talc (manufactured by Shokozan Mining Co., Ltd.), erythritol (manufactured by Nikken Chemical Industry Co., Ltd.), a flavoring agent (manufactured by Ogawa Perfumery Co., Ltd.) and magnesium stearate (manufactured by Kyodo Yakuhin K.K.) were added to the powders, followed by blending with a V-10 blender (manufactured by Tokuju Corporation) for 5 minutes. The blend was tableted with a pestle of 10 mm in diameter having flat-faced beveled edges using a tabletting machine but no tabletting problem was noted. The thus produced fast disintegrating tablets had a quality comparable to those obtained in EXAMPLE 12, indicating that trehalose is likewise available for the multilayered surface modification technique.

**TABLE 15**

| Formulation | |
|---|---|
| Component | Amount Added (unit: g) |
| Ibuprofen | 400 |
| Titanium oxide | 4 |
| Finely divided trehalose | 12 |
| Light silicic anhydride | 4 |
| Citric acid | 40 |
| Erythritol | 400 |
| Partially alphanized starch | 480 |
| Crystalline cellulose | 1000 |
| Flavoring agent (orange) | 12 |
| Magnesium stearate | 24 |
| Talc | 24 |
| Total | 2400 |

### TEST EXAMPLE 2

In order to explain the effects achieved by the present invention in more detail, tablet characteristics were determined with the fast disintegrating tablets of the invention obtained in EXAMPLES 10 through 13. The results are shown in TABLE 16. The results reveal that the fast disintegrating tablets obtained in the present invention are excellent in disintegratability and yet maintain a suitable strength. No tabletting problem (sticking to the pestle) was noted with the tablets obtained in EXAMPLES 10 to 13 and with the fast disintegrating tablets obtained in EXAMPLES 11 to 13, even the strongly stinging taste of ibuprofen was improved.

**TABLE 16**

| Example | 10 | 11 | 12 | 13 |
|---|---|---|---|---|
| Pestle | 10 mmφ, | flat-faced | beveled | edge |
| Sticking to pestle | none | none | none | none |
| Weight (mg) | 300 | 300 | 300 | 300 |
| Thickness of tablet (mm) | 3.5 | 3.5 | 3.5 | 3.5 |
| Diameter of tablet (mm) | 10.2 | 10.1 | 10.1 | 10.2 |
| Hardness (kg) | 7.6 | 8.5 | 7.1 | 8.8 |
| Disintegration time (1) | 23 secs. | 26 secs. | 22 secs. | 25 secs. |
| Disintegration time (2) | 70 secs. | 74 secs. | 70 secs. | 73 secs. |
| Taste (stinging) | yes | none^{*1} | none | none |

| | | | | |
|---|---|---|---|---|
| *1: When taken together with water, there was no stinging taste and the stinging taste was alleviated when taken without water. | | | | |

### Hardness:

The hardness is expressed as a mean value of five measurements as measured with Kiya type Hardness Tester.
(Hardness tester: HARDNESS TESTER (manufactured by KIYA))

### Disintegration time (1):

The disintegration time was measured with a disintegration tester specified in The Pharmacopoeia of Japan, 13th edition (no disk). The results indicate a mean value of six runs.
(Disintegration tester: table disintegration tester T-4H (manufactured by Toyama Sangyo Co., Ltd.))

### Disintegration time (2):

A sieve of 5.5 mesh was put in a beaker charged with 300 mL of water. One tablet was placed in the beaker. The total elapsed time required for the tablet to disintegrate, completely pass through the sieve and fall out of the mesh was recorded (distilled water: 23°C). The disintegration time is the average of the time recorded for 2 runs.

### INDUSTRIAL APPLICABILITY

The fast disintegrating tablets of the present invention can be produced by the dry method in an extremely simple way, without requiring any complex procedures, using conventionally available devices/ equipments. Moreover, the thus produced fast disintegrating tablets exhibit excellent dissolution and disintegration properties.

The fast disintegrating tablets of the present invention have a suitable disintegratability and solubility to be disintegrated to an appropriate size when holding water in the oral cavity so that the tablets are neither adhered to nor choked in the throat, and are easy for administration to aged or pediatric patients, and thus are advantageously used for the prevention or treatment of diseases in patients, particularly aged and pediatric patients.

The fast disintegrating tablets of the present invention have a sufficient strength and thus, are excellent for storage and stability over a long period of time. The fast disintegrating tablets of the invention yet maintain a strength sufficient to resist destruction upon administration, for example, when they are taken out of PTP packages. Therefore, the tablets of the invention can be advantageously used for the prevention or treatment of diseases in patients, particularly aged and pediatric patients.

Furthermore, by subjecting the pharmacologically active ingredients to surface modification using the surface modifying base material including light silicic anhydride, the pharmacologically active ingredient-comprising surface-modified powders having a good flowability can be obtained. The powders enable to produce the fast disintegrating tablets of the present invention by the dry production method.

Moreover, the multilayered surface modification technique by the dry coating in combination with the aforesaid surface modification using titanium oxide or erythritol having a mean particle size of 3 µm or less enables to produce tablets free of any tabletting problem and also enables to produce tablets with an improved taste.

## Claims

1. A surface-modified powder comprising a pharmacologically active ingredient and having a flowability enabling direct tabletting, said powder being surface-modified by subjecting a powder for surface modification comprising a pharmacologically active ingredient or a pharmacologically active ingredient and a diluent to surface modification with a surface modifying base material.

2. The surface-modified powder comprising a pharmacologically active ingredient according to claim 1, wherein the surface modifying base material is selected from members capable of physically adhering to the surface of the powder for surface modification and contributing to improving the flowability of the powder.

3. The surface-modified powder comprising a pharmacologically active ingredient according to claim 1 or 2, wherein the surface modifying base material is at least one member selected from light silicic anhydride, talc, stearic acid, magnesium stearate, calcium stearate, starch, titanium oxide, citric acid, malic acid, adipic acid, hydrous silicon dioxide and calcium carbonate.

4. The surface-modified powder comprising a pharmacologically active ingredient according to any one of claims 1 to 3, wherein the surface modifying base material is at least one member selected from light silicic anhydride, talc, stearic acid, magnesium stearate and calcium stearate.

5. The surface-modified powder comprising a pharmacologically active ingredient according to any one of claims 1 to 4, wherein light silicic anhydride is used as the surface modifying base material.

6. The surface-modified powder comprising a pharmacologically active ingredient according to claim 5, which contains 0.1 to 5 wt% of light silicic anhydride.

7. The surface-modified powder comprising a pharmacologically active ingredient according to any one of claims 1 to 6, wherein the pharmacologically active ingredient added with a diluent selected from lactose, erythritol, trehalose, anhydrous calcium hydrogenphosphate and crystalline cellulose has been surface-modified with the surface modifying base material.

8. The surface-modified powder comprising a pharmacologically active ingredient according to any one of claims 1 to 7, wherein the flowability is at most 42° in terms of an angle of repose.

9. The surface-modified powder comprising a pharmacologically active ingredient according to any one of claims 1 to 8, which is subjected to dry coating after adding at least one member selected from finely divided titanium oxide, talc, erythritol and trehalose to the powder for surface modification before or after the surface modification.

10. A method of producing the surface-modified powder comprising a pharmacologically active ingredient and having a flowability enabling direct tabletting according to any one of claims 1 through 9, which comprises blending, for surface modification, a powder for surface modification comprising a pharmacologically active ingredient and optionally further containing a diluent with a surface modifying base material.

11. A fast disintegrating tablet comprising the pharmacologically active ingredient-comprising surface-modified powder according to any one of claims 1 through 9, having blended with a disintegrant and directly tableted.

12. The fast disintegrating tablet according to claim 11, wherein partially alphanized starch or crospovidone is used as the disintegrant.

13. The fast disintegrating tablet according to claim 12, which contains 10 to 60 wt% of partially alphanized starch or crospovidone.

14. A method of producing the fast disintegrating tablet according to any one of claims 11 through 13, which comprises blending, for surface modification, a powder for surface modification comprising a pharmacologically active ingredient and optionally further containing a diluent with a surface modifying base material, adding a disintegrant to the blend and then subjecting the mixture to direct tabletting.

15. Use of the surface-modified powder comprising a pharmacologically active ingredient for producing a tablet by directly tabletting the surface-modified powder comprising a pharmacologically active ingredient according to any one of claims 1 through 9, optionally after blending the powder with an additive.

16. Use according to claim 15 for producing a fast disintegrating tablet.

17. A method of producing a tablet preparation, which comprises subjecting the surface-modified powder comprising a pharmacologically active ingredient according to any one of claims 1 through 9 to direct tabletting, optionally after blending the powder with an additive.

18. The method according to claim 17, wherein the surface-modified powder comprising a pharmacologically active ingredient is blended with a disintegrant to produced the fast disintegrating tablet.
